# EUROPEAN PATENT APPLICATION

(11) **EP 2 444 029 A1**
(43) Date of publication of application: **25.04.2012**
(21) Application number: 08776418.9
(22) Date of filing: 30.05.2008
(51) Int. Cl.: A61F 2/00

(54) **PREFORMED SURGICAL MESH**

(71) Applicant: Santos Bellas, Gabriel Antonio, Bogota N/A (CO)
(72) Inventor: Santos Bellas, Gabriel Antonio, Bogota N/A (CO)
(74) Representative: Lahidalga de Careaga, Jose Luis
(86) International application number: PCT/IB2008/052107
(87) International publication number: WO 2009/144532

(57) **Abstract**

This invention is related to the medicine branch, more exactly it is related to a prosthetic, preformed, non absorbable or mixed mesh with specific form and size. This is a T-cup mesh or T mesh-cup which is composed by two rectangular attachments with T-cup shape to be used in pelvic or urogynecological surgery in the treatment of:
Uterine prolapse: hysterocele.
Post-hysterectomy vaginal vault prolapse: cupolocele
Related or not to posterior enterocele or elongation of Douglas' bag bottom.
Related or not to middle or high rectocele
Related or not to posterior cystocele

## Description

### Background Art

T-cup mesh

Surgical meshes of synthetic material with different absorbable, non absorbable or mixed compositions, designed and based in a personal operative technique are presented since 1983. It was presented in the Medicine NationalAcademy in 1989 fro the treatment of uterus or vaginal vault prolapse. It final shape is a T- anterior to the uterine isthmus or vaginal vault level and with an image of posterior and open backward asymmetric 'cup' compressing the pelvic rectum at s2 and s3 level. It has an elongation in its right lateral extremity projecting towards up at level s-1 and I-5. It ends extending itself in oblique button shape attaching to the sacral promontory. After all these years of experience a new type of preformed mesh for urogynecological surgery is developed.

The use of mesh in surgery with the technological advent of plastics as the nylon and tergal as substitutes of the autologous products as the fascia lata and skin, homologous as the duramater or heterologous as the Oxfascia and bovine derivatives.

Synthetic meshes of absorbable constitution as the polyglactin which is substituted by the collagen and other derivatives of synthesis as the P.D.S and Maxon are more and more used in the contemporary surgery.

Non absorbable synthetic meshes manufactured with plastic derivative products by itself or combined with other absorbable products or the addition of antimicrobial products have been used for the closing of the abdominal wall defects as well as protrusions in the treatment of hernias, in urological surgery of urine incontinence and pelvi-gynecological surgery for defects of genital and rectal prolapses.

Basically these are crossing meshes of rectangular tissue and shape available in different sizes. Some of them may have ellipsoidal shape to adapt to the inguinal defects, other have small quadrilateral shape with anchor tapes for the treatment of incontinence, other ones have trapezoidal or triangular shape with anchor tapes fr the defects of the I, II and III partitions of the genital prolapses using the obturator artery, suprapubic or sacroespinous ligaments route.

These are mainly rectangular elongated portions of approximately 3cm width cut from the original rectangular mesh to make fixations to the ligaments of the pelvic as sacroespinous,in special to the sacral promontory anchoring the vaginal vault or the uterus to these fixed elements to give them support in case of uterus or post-hysterectomy vaginal vault prolapses. Habitually, such mashes are fixed to those elements through several suture points either by laparotomy pelvic-laparoscopic route.

The new preformed prosthetic mesh presented below may be manufactured from any non absorbable or absorbable material or the mixture of both.

It has a spatial, new and original T-cup shape with a right lateral elongation in palette or terminal button shape

Its design is adapted from a surgical technique created by Dr. Gabriel Santos Bellas in 1983 and presented in the Medicine NationalAcademy of Venezuela on January 14 th 1989 and in different congresses and surgery and gynecological journeys with more than 23 years up to now (2007).

The original surgical technique for the treatment of uterine or post-histerctomy vaginal vault prolapse associated or not to enterocele,was called histerocolpopexia of sacro uterine ligaments with fixation to the sacral promontory where number 0 or 1 absorbable material or non absorbable material may be used in accordance with the age and parity either desired or not by the patient.

It begins with the sacral promontory as initial and final point of the surgery and the continuous suture or surget displaces from the retro-peritoneum to the pelvic cavity with 3cm downward and it will be threaded in a free edge for several entries and exits of the right uterosacralligament, totally, until reaching the insertion of the uterine isthmus from this or in lack of this to the right margin posterior face of the vaginal vault. Two anchorages of the suture are made at isthmus level or the vaginal vault at right and left level.

Then the suture goes to take the left uterosacralligament in its halfway making 2 to 3 contra-lateral points with the right uterosacral and this way a 'T' shape will be formed by pulling the surget. Such 'T' is put in ateroposterior direction transversally laid down with the 'T' horizontal branch at level of the uterus or vaginal vault and the longitudinal branch behind it pointing towards the pelvic rectum.

The suture anchors the sero-muscular of the rectum in its anterior and right lateral size, it retakes the posterior part of the right uterosacralnear to its origin in the posterior insertion and ascends backward approximately 3cm reaching to the anchorageinitial point in the sacral promontory. This going to produce a 'cup' shape or posterior concavity that embraces the rectum contour without compressing it, closing this way the Douglas' bag bottom by it taller level and obliterating this way the entorecele, if any, it avoids the production of internal hernias and suspends the uterus or the vagina following its s-3 s-4 normal physiological axis.

The 'pallet' or 'button' shape is due to the posterior and superior angle of the suture both when it exits as well it returns from the promontory level at 3cm taller than the uterosacrallevel. Due to this, from the concave and put in transversal form of posterior and asymmetric opening 'cup', and for being the right hemicup-rightuterosacral larger than the left hemicup- half of the left uterosacral, two anchoragesutures are unpinned upward and backward at the level of the sacral promontory starting fro the larger posterior edge of the right sacral uterus and that figuratively can be compared to the 'stick-cherry' of the cup of a cocktail or ice remover.

This is the shape of the design originated from the original surgical technique of Dr. Gabriel Santos and which implied the creation of prosthetic material as the 't-cup' mesh with fixation to the sacral promontory an with all the advantages of the original surgical technique plus the greater resistance and stability of the axis or framework of the suspension 't-cup' preformed mesh.

The principles of the original surgery as the initial and final anchorageto the sacral promontory and the closing of the Douglas' bag bottom are maintained by the suture of the uterosacraland the anchorageof the prolapsed organ uterus and/or vaginal vault to a resistant point.

The maintenance or preservation of the vaginal axis oriented to s3-s4 and on the other hand a vaginal longitude of 7mc leaving the space of the sacral-subpubic pelvic diameter that has a longitude of 12cm and preserving the posterior 5cm for the putting of the preformed 't-cup' mesh.

The preformed placation of the axis or framework of the 't-cup' mesh due to its major thickness give a better resistance, the anchoragepoint is of major resistance in the pelvis in accordance with the study of resistance of the pelvis performed in corpses by Lazarou G. Et al (2004).

The axis or framework of the t-cup mesh has a vertical sagital axis with a thickness of 0.15cm when it is three-laminar and of 0.2cm when it is tetralaminar with a height or profile of 1.5cm and a similar width in cm from the horizontal branch from the 't' to the 'cup'.

In the anterior extremity of the quadrilateral shape if forms in turn an angle of 90° with the vertical branch of the 'T', this quadrilateral measures 1.5cm of width x 2.5 cm length forming the anterior part of the 'T'. This part will be sutured to the uterine itsmo or the superior part of the vaginal vault.

In its anterior extremity two double laminates of 0.1cm width are attached prolonging upward or backward from the above mentioned 't' quadrilateral. The first one has 2.5cm of width for the fixation of the vaginal vault (anterior wall) in the inter-vesical-vaginal space with a length of 3cm, the inferior or posterior one that leaves arista diedra downward from the origin of the 't' has a bi-laminar laminate of 0.1cm with a width of 2.5cm that along with the anterior or superior one form the image of the horizontal 'T' of the 't-cup' mesh. This posterior branch has a length of 6cm and may be adaptable to the posterior vaginal wall or to the rectum or the vaginal-rectal space. If any mesh remains, if can be sectioned to the measure or fold backward on the rectum anterior face.

Continuing from the 't' vertical branch, a 'cup' is formed which is concave backward, the profile or height remains of 1.5cm. The left size ends rounded with 2.5 of length while the right size is larger with 3.5cm.

The cup internal diameter is of 3.5cm to embraces, without comprising the rectum.

In the posterior extremity or the right final edge of the 't-cup' mesh the sagital axist of 1.5cm (right lateral) is folded and it continues with a inclination upward among 135degress and 145 degrees and with a length of 3cm with the same thickness than the framework of the tri-laminar or tetralaminar mesh (0.15 to 0.2cm) as the case may be. This folded or preformed stem is 1.5 width x 3cm length and bends backward in its final extremity and upward with an angle of 135 degrees and 145 degrees where it enlarges from 2cm length to 1.4cm width in rebounded shape as a 'button' or 'palette' to be fixed in the sacral promontory. Taking into account the pluripathology observed in the genital prolapses, cystocele and rectocele the support axis of the t-cup mesh is added with a complement conformed by two double laminar anterior prolongations (fold laminate sheet), with an short anterior or superior prolongation for the vaginal vesical space of approximately 3.cm length x2.5cm width and join the enlarged or enterocele rectovaginal space.

The anterior prolongation is adapted to the preferences of the surgeon if an anterior and posterior anchorageis desired in the vaginal vault prolapses or by sectioning the anterior or superior sheet if there is a uterus prolapse or if only a posterior vault anchorageis desired.

The length of the posterior or inferior sheet permits a larger anchorage with the simultaneous closing of the Douglas' bag bottom being able to fix it to the vaginal rectum space and/or the rectum anterior wall if the surgeon desires it or only by sectioning the mesh to the esteemed large for the required or available vaginal-rectum space.

The shape or proform of the 't-cup mesh' is asserted as an innovation at world level for meshes in the treatment of uterus genital and vagina vault prolapses either with or without cystocele, rectocele and/or enterocele.Its width, tackiness or angles can be modified by the 't-cup' shape with right posterolateral prolongation and the kinking ledge of fixation to the sacral promontory is original invention of medical doctor and surgeon Gabriel Antonio Santos Bellas.

**Advantages of the application of the 't-cup mesh'**

Anchorage point

The sacral promontory is an anterior common vertebral ligament, is the more resistant zone of the pelvis according the studies of resistance to pressure performed on corpses by Lazarou G. Et al (2004).

Limits minor pelvis: uterosacral ligaments: enterocele

The superior limit of the pelvis and the enterocele to be obliterated is a free superior edge level of the uterosacralligaments and therefore, of any posterior enterocele related to the uterus or vaginal vault prolapses, specially those produced after applying techniques of urine incontinence correction by retro-pubic route- M.M,K, Burch and its modifications as well as the supra-pubic route and also after vaginal hysterectomies by the shortening and verticalization of the vaginal axis after a vaginal hysterectomy.

Restoration of the normal vaginal axis

The use of the edge of the uterosacralligaments and fix them to the 't-cup mesh' as well as the fixation anterior to the uterine isthmus in cases of hysterocele or the vaginal vault in the vaginal vault prolapses after a hysterectomy, a stable and fixed suspension to the prolapsed utero vaginal is produced following the normal vaginal axis to the s3-s4 vertebras.

Vaginal longitude and pelvic diameters, space for the 't-cup mesh' placing.

This suspension is made taking advantage of the sub-public middle sacral pelvic diameter of the pelvis which is of 12cm length and which leaves a intrapelvic vagina of 7cm and a posterior space of standard 5cm in which the 't-cup mesh' going to be fixed around the rectum without compressing it.

This way overcorrections of the vaginal large are avoided and in special the loss of the uretrovesical posterior angle.

In case of hysterocele in a 30% related to retro deviations, this pathology is corrected by returning the uterus its physiological and functional anteversion face to the efforts and by reestablishing the cut of these uretrovaginal physiological axes and at the same time the support of the uterine neck on the elevator raphe face to the efforts and bidepestation.

Maintenance of the rectal intestinal traffic and prevention of internal hernias

The 'cup' shape of 3.5 cm of diameter open backward (towards the sacral) permits to adapt itself to the tubular convexity of the rectum without compressing it and fix it in the ¾ of its anterolateral circumference using the half of the left uterosacral and leaving a left posterior space at the flap sacral level and the body of the sacrum and, on the other hand, giving the totality of the right uterosacral, without altering the intestinal traffic which have been verified through rectoscopies.

Fixation to the more resistant point to pressures in the pelvis

The posterior and superior disposition of the right lateral prolongation of the 't-cup mesh' is approximately of 3cm and directs the traction and supports the visceral weight when the patients is upstanding towards the resistant fixation point which is the sacral promontory. The mesh shape and the thickness of the 't-cup' transversal axis triplicate or quadruplicate the resistance factor of this one face to pressures and the intra-abdominal effort; likewise the pressure forces are shared in the pelvic contour of its anterolateral fixation of the uterosacralligaments and the remain of the vesical-vaginal endopelvic fascia.

Prevention and treatment of enteroceles

After placing the 't-cup mesh', no spaces for the appearance of enterocelesare leaved, the Douglas' bag bottom is shortened by the posterior and inferior fixation of the mesh and therefore it becomes taller at the pelvis superior strait. The pelvic surface is upholstered or covered by the pelvic peritoneum of the uterosacralligaments to which it was fixed. On the other hand, the inferior prolongation of the rectangular mesh can be fixed in the enlarged bottom of the Douglas' bottom which conforms the enterocele or the superior rectovaginal space up to 6cm. This inter-vaginafixation of the 't-cup mesh' shortens it or folds the inferior prolongation on the rectum anterior face and then it is fixed to the posterior face of the vagina or the rectum anterior.

The physiological vaginal axis is not altered, maintaining the projection of the t-cup mesh transversal axis to the s-3 s-4 uterosacralinsertion. The bilateral dissection of ligaments and the sacroespinous muscles are not necessary as other type of mesh placing.

**Bibliographical Reference**

Types of Mesh and Companies Producers of Meshes for the surgery of genital prolapse and urine incontinence.

American Medical Systems (AMS), Monarc™ SubfascialHammock â, SPARC™ Sling System â, http://www.americanmedicalsystems.com,

Johnson&Johnson â Gateway â, Pelvic Organ Prolapse Using the TVM Technique, Trans-Vaginal Mesh (TVM) Technique, GYNECARE â PROLIFT Pelvic Repair Systems Labeling, GYNECARE â PROLIFT For Pelvic Organ Prolapse, http://www.jnjgateway.com

Mentor Corporation. , Salzano L, Rota G, Bellini S, D'Afiero A,. Uratape: short term results of a prospective multicentric study. [Eur Urol. 2004] PMID: 15183554 [Analysis of complications of the tension-free vaginal tape procedure for surgical treatment of female stress urinary incontinence], [Ginekol Pol. 2003] PMID: 14674147 Tension-free transobturator approach for female stress urinary incontinence, Transobturator tape (Uratape): a new minimally-invasiveprocedure to treat female urinary incontinence. [Eur Urol. 2004] PMID: 14734007, [Transobturator tape (Uratape). A new minimally invasive method in the treatment of urinary incontinence in women] [Prog Urol. 2003] PMID: 14650298 Surgical treatment of female stress urinary incontinence with a trans-obturator-tape (T.O.T.), [Prolene mesh sling in the treatment of stress urinary incontinence. Integral treatment of pelvic floor anomalies. Long-term results] [Arch Esp Urol. 2002] PMID: 12564066, http://ir.mentorcorp.com.

Sofradim International/Bard, URETEX® (treatment of female stress urinary incontinence), PARIEFIX® (laparoscopic mesh fixation), PARIETEX® (treatment of female prolapse), http://www.sofradim.com

Tyco, Obturator IVS Tunneller™,http://www.tycohealth-ece.com

Bibliographical Reference about use surgical of the different meshes

Galmés Belmonte; E. Días Gómez. Comunicación especial: ¿Son iguales todos los sistemas empleados paracorregir la incontinencia urinaria mediante mallas libres de tensión? Actas Urológicas Españolas v.28 n.7 Madrid jul.-ago. 2004

Schmidbauer, S; Ladurner, R; Hallfeldt, K; Mussack T., Heavy Weight vs Low Weight Polypropylene Meshes for Open Sublay Mesh Repair of incitional Hernia. Eur J Med Res (2005) 10:247-253 - June 22 2005BIRCH

C, FYNES MM, The role of synthetic and biological prostheses in reconstructive pelvic floor surgery. Curr Opin Obstet Gynecol 2002a oct; 14 (5): 527-535.

AMID PK, SHULMAN AG, LICHTENSTEIN IL, HAKAKHA M., Biomaterials for abdominal wall hernia surgery and principles of their applications. Langenbecks Arch Chir 1994; 379 (3): 168-171.

WELTY G, KLINGE U, KLOSTERHALFEN B, KASPERK R, SCHUMPELICK V. Functional impairment and complaints following incisional hernia repair with different polypropylene meshes. Hernia 2001 sep; 5 (3): 142-147.

COSSON M, BOUKERROU M, LOBRY P, CREPIN G, EGO A., Mechanical properties of biological or synthetic implants used to treat genital prolapse and stress incontinence in women: what is the ideal material?. J Gynecol Obstet Biol Reprod (Paris) 2003 jun; 32 (4): 321-328.

DIETZ HP, VANCAILLIE P, SVEHLA M, WALSH W, STEENSMA AB, VANCAILLIE TG., Mechanical properties of urogynecologic implant materials. Int Urogynecol J Pelvic Floor Dysfunct 2003 oct; 14 (4): 239-243; discussion243.

FALCONER C, SODERBERG M, BLOMGREN B, ULMSTEN U., Influence of different sling materials on connective tissue metabolism in stress urinary incontinent women. Int Urogynecol J Pelvic Floor Dysfunct 2001; 12 Suppl 2: S19-S23.

DAHER N, BOULANGER JC, ULMSTEN U., Prepubic TVT: an alternative to classic TVT in selected patients with urinary stress incontinence. Eur J Obstet Gynecol Reprod Biol 2003 apr 25; 107 (2): 205-207.

DEVAL B, LEVARDON M, SAMAIN E, RAFII A, CORTESSE A, AMARENCO G, CIOFU C, HAAB F., A French multicenter clinical trial of SPARC for stress urinary incontinence. Eur Urol 2003 aug; 44 (2): 254-258; discussion 258-259.

DELORME E., Transobturator urethral suspension: mini-invasive procedure in the treatment of stress urinary incontinence in women. Prog Urol 2001 dec; 11 (6): 1306-1313.

DE LEVAL J., Novel surgical technique for the treatment of female stress urinary incontinence : transobturator vaginal tape inside-out. Eur Urol 2003 dec; 44 (6): 724-730.

ULMSTEN U, HENRIKSSON L, JOHNSON P, VARHOS G., An ambulatory surgical procedure under local anesthesia for treatment of female urinary incontinence. Int Urogynecol J Pelvic Floor Dysfunct 1996; 7 (2): 81-85; discussion85-86.

BEZERRA CA, BRUSCHINI H.,Suburethral sling operations for urinary incontinence in women.Cochrane Database Syst Rev 2001; (3): CD001754.

Bibliographical Reference about the use of homologous materials, autologous materials, heterologous materials and synthetic materials (meshes)

Lazarou, G. Scotti, R et al., Pullout strengths of sacral an vaginal attachment sites in Cadavers. J of Pelvic Medicine & Surgery 10(4):209-212 July/August 2004.

Bethoux, A., Anatomía Funcional Cap XXXI, en : Robert, H.G. Tratado de Técnica Quirúrgica Tomo XIV. 19 ed. Barcelona, Toray, Masson. S.A.1972.

Falk, H.C., Uterine Prolapse and prolapse of the vaginal vault treated by sacropexy. Obstet. Gynecol. 18 (1): 113-115. (1961)

Te Linde, R.W.: Mattingly, R.F. & Thompson, J.D., Ginecología Operatoria. Editoral'El Ateneo', Buenos Aires. Sexta Edición. (1987)

Te Linde, R. W. Cap XXIV, Malposiciones del Utero Muñon cervicaly Vagina. Prolapso de Utero. Prolapso de la Vagina Secundario a la Histerectomía. Ginecología Operatoria. Editoral'El Ateneo', Buenos Aires. Sexta Edición. (1987)

Ridley John Cap XXV, Uerocele, Cistocele e incontinencia de orina al esfuerzo. Ginecología Operatoria. Editoral'El Ateneo', Buenos Aires. Sexta Edición. (1987)

Ridley John Cap XXV, Incontinencia urinaria no curable por la Plicatura del esfínter. Ginecología Operatoria. Editoral'El Ateneo', Buenos Aires. Sexta Edición. (1987)

Symmonds, R.E., Reparaciones de los Soportes Pélvicos. En Benson, R.C. Diagnóstico y Tratamiento gineco-obstétrico. Ed. El Manual Moderno S.A. México, 1979:250-267.

Arthure, H.G.& Savage, D, Uterine Prolapse and Prolapse of de vaginal vault treated by sacral hysteropexy. J. Obstect. Gynecol. Br. Emp. 64: 355-360. (1957)

Yates, M J., An Abdominal approachto the repair of posthysterectomy vaginal inversion. Br J Obstec. Gynec. 82: 917-819 (1975)

Rust, J. A.; Botte, J M and Howlet, E J., Prolapse of the vagina vault. Am J. Obstet Gynec. 125(6):768-776 (1976),

Ridley, J H., A composite vaginal vault suspention using fascia. Am J. Obstet Gynec. 126(6):590-596 (1976)

Lane, F E., Modifec Technique of sacral colpopexy. Am J. Obstet Gynec. 140:836 (1981)

Thornton, W N & Peters, II W A., Repair of vaginal Prolapse after Hysterectomy. Am J. Obstet Gynec.147140-148 (1983)

Lansmann,H H., Post-Hysterectomy vault prolapse sacral colpopexy with dura mater graft. Am J. Obstet Gynec. 63: 577-581 (1964)

GrundsellH & Larsson, G., Operative management of vaginal vault prolapse following Hysterectomy., Br J. Obstet Gynec. 91:608-611 (1964)

Kauppila O. Punnonen R. & Teissala K., Prolapse of the vagina after Hysterectomy. Surg Gynec Obstet 161:9-11 (1965).

### Description of Drawings

As a way of illustrating this invention and in order to clarify the descriptive memory, a set of figures detailing to explicative and not limitative way the preformed mesh as result of the invention are attached herein:

Figure 1: Superior view

Figure 2: Inferior view

Figure 3: Right lateral view

Figure 4: Left lateral view

Figure 5: Anterior view

Figure 6: Posterior view

Figure 7: Right lateral view, with formative elements of the cup fold for the application of the vaginal vault prolapse and placed for the anterior-posterior fixation.

Figure 8: Right lateral view, with formative left element of the cup omitted for the application of the vaginal vault prolapse remaining only the right element placed for the posterior fixation.

Figure 9: Right lateral view, with formative left element of the cup omitted for the application of the vaginal vault prolapse remaining only the partially sectioned right element placed for the posterior fixation.

Figure 10: Right lateral view, with the formative elements of the cup totally sectioned for the application of the uterine prolapse, remaining placed for the fixation in the uterine isthmus (hysterocele)

Figure 11: Anatomy drawing illustrating the intrauterine location of the preformed mash with all the elements composing it.

Figure 12: It shows in perspective the different types of preformed mesh starting from the most universal (12a), the most particular (12b, 12c, 12d) by selecting some of its elements.

In each figure can be observed that the surgical mesh embracesa thermal-coagulated or not preformed element, that can be bi, tri or tetralaminar according the type of mesh to be built; the thickness of said mesh may ranks between 0.10 and 0.20cm.

The horizontal anterior part (1) and the vertical central segment (2) can be of a thickness double than the quadruple of thickness of the original laminate; then towards the cup shape posterior part each hemicup (1a, 1b) is bi-laminar, the left hemicup (1a) is shorter to be placed in the para-rectal region and ends in the bisel; the right hemicup (1b) folds on itself and ascends vertically from 2 to 3 cm changing from bi-laminar to tetralaminar;it bends backward approximately 1.5 to 2cm of length x 1.4cm of width in palette of button shape (5).

The anterior segment (1) has rectangular shape and its ideal dimensions can be from 2.5 to 3.5 cm of length x 1.5 to 2cm of width, it can be conformed by bi-laminar elements superior and inferior from 4 to 6 cm of length.

The vertical branch (2) of the 'T' must measure 1.5cm of length as maximum, maintaining a width of 1.5 to 2cm; each hemicup has 1.5 to 2cm of width x 3 to 3.5 cm of length.

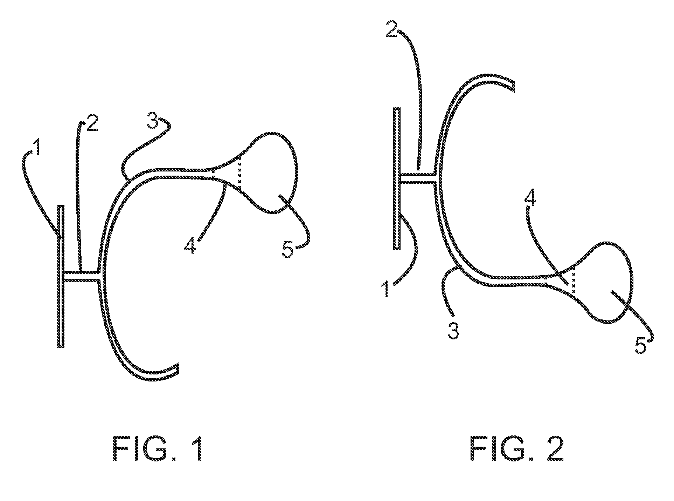

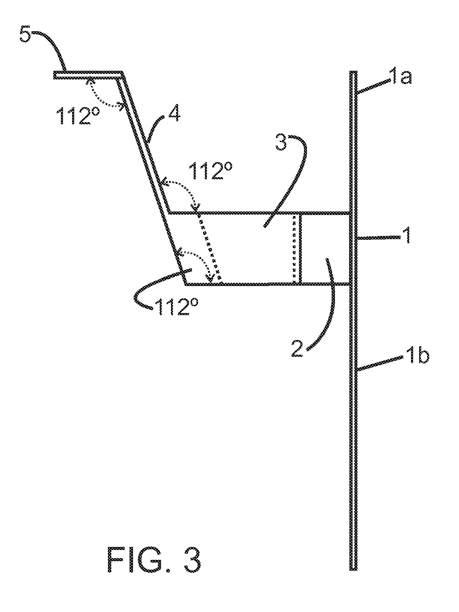

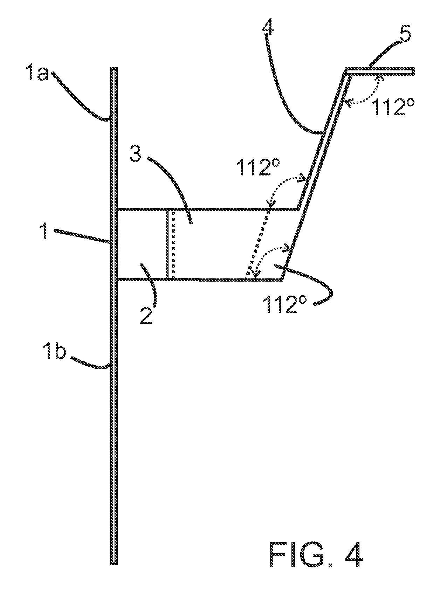

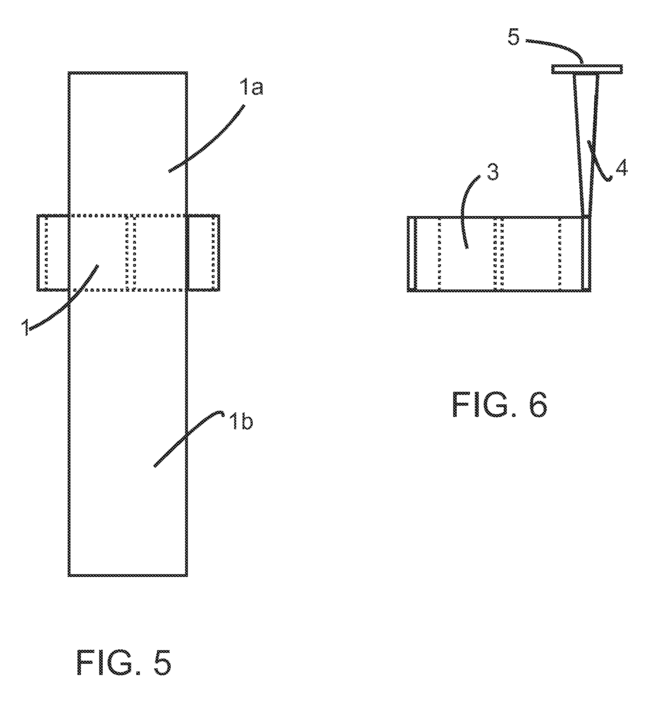

In figures 1 and 2 it can be observed the preformed mesh showing said mesh from superior and inferior views. Figures 3 and 4 are the right lateral and left lateral views where elements 1, 1a and 1b conforming a rectangular laminate such as corresponds to the mesh preformation mesh where figures 5 and 6 can be seen in detail in their corresponding anterior and posterior views.

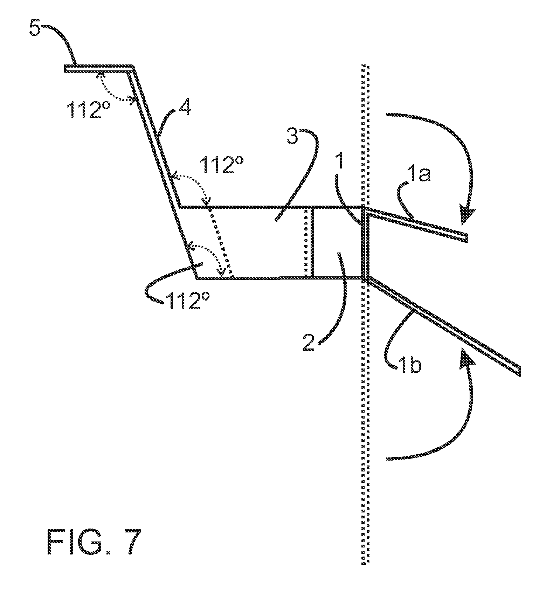

Figure 7 is a right lateral view of the most universal mesh as result of the invention where the rectangular element folds to form a cup with the left (1a) and right (1b) elements joined through a central element (1); as can be seen in such figure the left element (1a) is shorter than right element (1b) and they are fold in such a way that they adapt with a surgical mesh for the correction of the vaginal prolapse. Hemicup (1a and 1b) as shown, permit the previous-posterior fixation within the pelvis. The left element (1a) permit the previous fixation within the pelvis and the right element (1b) permit the posterior fixation within the pelvis.

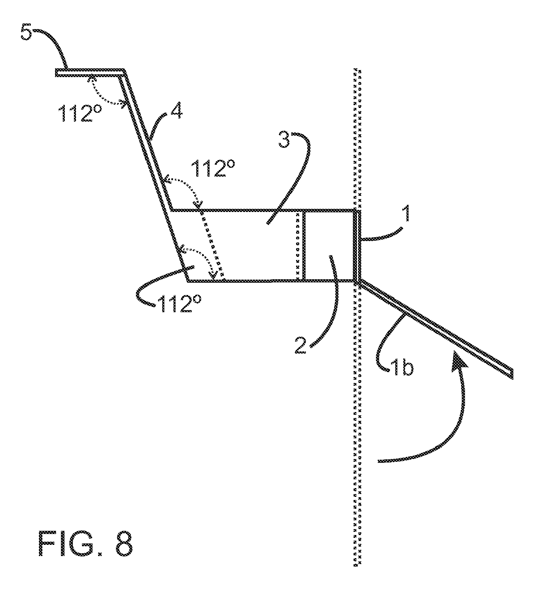

Figure 8 is a right lateral view of the preformed surgical mesh where the rectangular element forming the cup has sectioned the left element (1a) with regards to figure 7 remaining only the right hemicup (1b) which folds following the central element (1); this mesh as shown, adapts for being used in the correction of the vaginal vault prolapse with posterior fixation only to the sacral promontory.

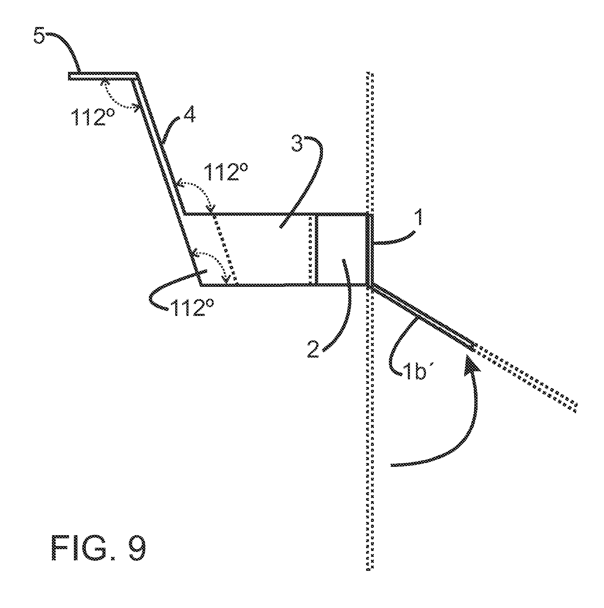

Figure 9 is a right lateral view of the preformed surgical mesh where the rectangular element forming the cup has totally sectioned the left element (1a) and partially sectioned the right element (1b) remaining the mesh with the 1b hemicup which adapts for the operations of vaginal prolapse for cases of short Douglas' bag bottom.

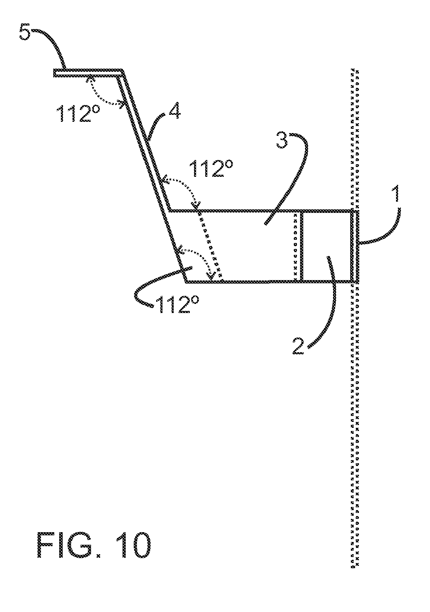

Figure 10 is a right lateral view of the preformed surgical mesh where elements 1a and 1b are totally put aside remaining only the central element (1) which anatomically adapts to the operations of the correction of uterine prolapse where the element of central fixation is the uterine isthmus (hysterocele).

In figures 7, 8 , 9 and 10 can be observed the element 3 which is a circular shell of uniform width which adapts to the utero-vaginal cavity and of which one of its extremities has a prolongation is askance (4) in which extremity other element bends in button or palette shape (5) and which permits the fixation of the mesh to the intrauterine cavity walls.

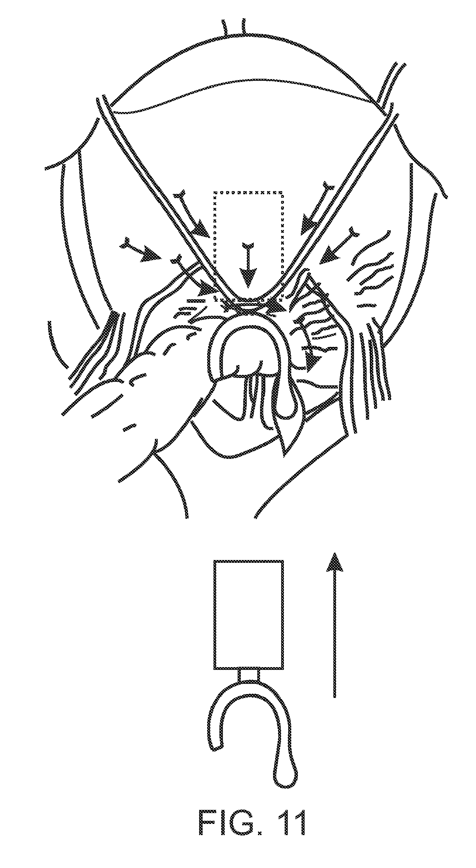

Figure 11 is a illustration of how the preformed mesh adapts within the intrauterine mesh choosing for this case the most universal preformed mesh as an example of application of this invention.

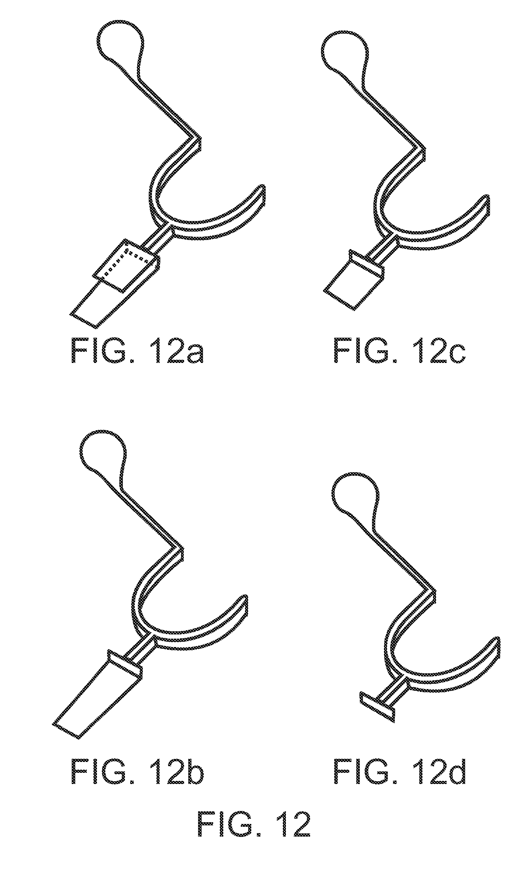

Figure 12 shows in isometrics each one of the preformed meshes starting from the most universal (12a) to its most common variants 12b, 12c and 12d such as its use is described in corresponding figures 7, 8, 9 and 10.

## Claims

1. Surgical, thermal-coagulated or not, non absorbable or mixed prosthetic mesh which shape and size is a T cup mesh with two rectangular prolongations that fold on another central one for the adaptation to pelvic-urogynecological surgery. Such mash besides of having a central concavity in one of its extremities that raises in inclined ascent another trapezoidal element in which other bend extreme a palette or button shape.

2. Surgical, thermal-coagulated or not mesh according to assertion 1, characterized because this mesh can be bi-laminar, tri-laminar or tetralaminar with an approximate thickness of 0.10cm and 0.20cm for each laminate.

3. Surgical, thermal-coagulated or not mesh according to assertion 1, characterized because the cup formed starting a rectangular is bi-laminar with the central region with dimensions between 2.5cm to 3.5cm of length and 1.5cm to 2cm of width. From such central region the rectangle folds or doubles to form a left hemicup that measures between 3cm and 4cm cm of length and between 2.5cm and 3.5cm of width; the right hemicup formed from the same rectangle measures between 4cm and 6cm of length and 2.5cm and 3.5cm of width.

4. Surgical, thermal-coagulated or not mesh according to assertion 1, characterized because the vertical branch of the T must have as maximum 1.5cm of length and between 1.5cm of length and 1.5cm and 2cm of width.

5. Surgical, thermal-coagulated or not mesh according to assertion 1, characterized because starting from the vertical branch of the T originates a concavity with a diameter of 3.5cm and 1.5cm to 2cm of width.

6. Surgical, thermal-coagulated or not mesh according to assertion 1, characterized because in the posterior extremity or final of the right edge a satellite axis that folds to continue with an inclination upward between 112 and 140° of inclination enlarging itself 2cm of length x 1.4cm of width in rounded shape as button or pallet.

7. Surgical, thermal-coagulated or not mesh according to assertion 3, characterized because when the left hemicup of the mash sections it anatomically adapts for a correction of vaginal prolapse for posterior fixation to the sacral promontory.

8. Surgical, thermal-coagulated or not mesh according to assertion 3 characterized because by totally sectioning the left hemicup and partially sectioning the right hemicup of the mash it adapts for corrections of vaginal vault prolapse for posterior fixation with short Douglas' bag bottom.

9. Surgical, thermal-coagulated or not mesh according to assertion 3, characterized because totally sectioning the left and right hemicup only remains the central region and the mash adapts for corrections of uterine prolapse for the fixation to uterine itsmo (hysterocele).
